Europäisches Patentamt

European Patent Office

Office européen des brevets

(19)

(11) Veröffentlichungsnummer: **0 161 009**
**B1**

(12) # EUROPÄISCHE PATENTSCHRIFT

(45) Veröffentlichungstag der Patentschrift:
**28.12.88**

(21) Anmeldenummer: **85200109.8**

(22) Anmeldetag: **01.02.85**

(51) Int. Cl.⁴: **C 07 D 307/92**

(54) **Verfahren zur Herstellung von Naphtholacton.**

(30) Priorität: **10.05.84 DE 3417278**

(43) Veröffentlichungstag der Anmeldung:
**13.11.85 Patentblatt 85/46**

(45) Bekanntmachung des Hinweises auf die Patenterteilung:
**28.12.88 Patentblatt 88/52**

(84) Benannte Vertragsstaaten:
**CH DE FR GB LI**

(56) Entgegenhaltungen:
**FR-A- 2 488 608**

(73) Patentinhaber: **RÜTGERSWERKE AKTIENGESELLSCHAFT, Mainzer Landstrasse 217, D-6000 Frankfurt am Main 1 (DE)**

(72) Erfinder: **Talbiersky, Jörg, Dr., Neckarstrasse 7, D-6806 Viernheim (DE)**
Erfinder: **Pingen, Heinz, Alexander-Strasse 35, D-4100 Dulsburg 12 (DE)**
Erfinder: **Langenkamp, Martha, Siegmundstrasse 5, D-4200 Oberhausen 11 (DE)**
Erfinder: **Schäferling, Heinz, Hülskathstrasse 19, D-4200 Oberhausen 11 (DE)**

## Beschreibung

Die Erfindung betrifft ein Verfahren zur Herstellung von 8-Hydroxy-(1)naphthoesäure-lacton. Diese Verbindung wird als Naphtholacton bezeichnet.

Naphtholactone werden zur Synthese von Naphthofuranfarbstoffen benutzt, die aufgrund ihrer pH- und temperaturabhängigen optischen Eigenschaften für die Temperaturmessung und -regelung industrieller Anlagen eingesetzt werden oder für die Herstellung von druck- oder wärmeempfindlichem Kopier- oder Registriermaterial Verwendung finden.

Aus DE 3 126 057 C2 ist ein Verfahren bekannt, Naphtholacton durch thermische Behandlung von Naphthoesäure-(1) und Kupfer(II)-oxid herzustellen. Desgleichen ist aus FR-A-2 488 608 bekannt, Naphtholacton durch Thermolyse von Kupfer (II)-naphthoat bei Temperaturen oberhalb von 200 °C, bevorzugt von 250–280 °C herzustellen.

Bei dieser an sich einfachen thermolytischen Synthese laufen aber Zersetzungsreaktionen unter Bildung harzartiger Nebenprodukte ab. Aber nicht nur diese unerwünschte Harzbildung, die bis zu 16% des Umsatzes beträgt, beeinträchtigt die Wirtschaftlichkeit des Verfahrens, sondern auch die relativ hohe Umsetzungsrate zu Naphthalin.

Es ist daher Aufgabe der Erfindung, ein einfaches Verfahren zur Herstellung von Naphtholacton aus Naphthoesäure-(1) bzw. ihrem Kupfer (II)-Salz zu finden, bei dem möglichst keine harzartigen Nebenprodukte anfallen, d.h. bei dem eine möglichst hohe Umwandlungsrate der Naphthoesäure in Naphtholacton bzw. andere hochwertige Naphthoesäurederivate erfolgt.

Die Lösung der Aufgabe erfolgt durch ein Verfahren gemäss der Patentansprüche 1 bis 4.

Es wurde gefunden, dass bei dieser erfindungsgemässen Kombination von Reaktionskomponenten eine Lactonbildung bei relativ milden Reaktionsbedingungen erfolgt, wobei nur geringe unerwünschte Harzbildung auftritt. Somit entstehen bei diesem Verfahren in hohem Masse wirtschaftliche verwertbare Reaktionsprodukte.

Die Lactonausbeute erreicht 55%. Der ebenfalls gebildete Naphthoesäure-(1)-naphthylester fällt mit einer Ausbeute von unter 15% an. Die Summe aus Ester und Lactonausbeute kann bis zu 69% betragen. Es ist ein weiterer Vorteil dieses Verfahrens, dass auch die Bildung von Naphthalin wesentlich zurückgedrängt ist zugunsten der höherwertigen Produkte Lacton und Naphthoesäurenaphthylester. Die Umsätze liegen bei Reaktionstemperaturen zwischen 160–220 °C bei 60–85%.

Nicht umgesetzte Naphthoesäure kann durch einfache Extraktion mit Alkalibicarbonatlösung aus dem Reaktionsprodukt entfernt und in einem neuen Reaktionsansatz wieder eingesetzt werden.

Ausgangsprodukt für das erfindungsgemässe Verfahren ist reine oder technische Naphthoesäure-(1).

Als Kupfer wird ein möglichst feinteiliges, handelsübliches Kupferpulver eingesetzt in einer Menge von etwa 2 Mol Kupfer pro Mol Naphthoesäure-(1). Eine geringfügige Abweichung von etwa ±0,2 Mol bewirkt keine wesentliche Ausbeuteverschiebung. Eine wesentlich geringere Kupfermenge bedingt eine nicht ausreichende Umsetzung, während eine höhere Kupfermenge die umsatzbezogene Lactonausbeute verschlechtert.

In einer Variante des erfindungsgemässen Verfahrens wird anstelle eines Gemisches aus Kupfer und Naphthoesäure Kupfer(II)-naphthoat eingesetzt. Dadurch sind Umsätze von 80% und eine Lactonausbeute von 60% erreichbar. Die Ausbeute an Naphthoesäure-(1)-naphthylester liegt je nach Reaktionsbedingungen zwischen < 1 und 8%. Die Summe aus Ester- und Lactonausbeuten erreicht Werte von 67%. Damit ist die Lactonselektivität bei vergleichbaren, bzw. grösseren Umsätzen deutlich höher als bei Anwendung des in DE 31 26 057 C2 beschriebenen Verfahrens.

Die dritte Reaktionskomponente ist ein am Kern halogensubstituierter aromatischer Kohlenwasserstoff oder ein Gemisch derartiger kernhalogenierter aromatischer Kohlenwasserstoffe.

Beispiele für derartige kernhalogenierte Aromaten sind Chlorbenzol, Brombenzol, Dichlorbenzol, Chlortoluole, Dichlortoluol, Chlorxylole, Dichlorxylole, Hexachlorbenzol.

Es wurde allerdings gefunden, dass alkylsubstituierte Halogenaromaten die Reaktion in stärkerem Masse zur Veresterung der Naphthoesäure dirigieren.

Gute Lactonselektivitäten und Umsätze werden mit mehrfach kernchlorierten Benzolen erzielt. Einige dieser Halogenaromaten sind vergleichsweise billige grosstechnische Produkte.

Besondere Vorteile ergeben sich dadurch, dass Stellungsisomerie und Halogenierungsgrad bei den kernhalogenierten Benzolen ohne wesentlichen Einfluss auf den Erfolg des erfindungsgemässen Verfahrens sind. Aus diesem Grunde können die unterschiedlichsten Gemische kernhalogenierter Benzole Verwendung finden.

Die durch den Halogenaromaten eingebrachte Halogenmenge soll mindestens der doppelten molaren Menge der eingesetzten Naphthoesäure entsprechen. Da aber ein Überschuss an Halogenaromat zu einer beschleunigten Umsetzung führt und keine störenden Nebenreaktionen auftreten, und da sich die nicht umgesetzten Halogenaromaten leicht wieder zurückgewinnen lassen, wird diese Reaktionskomponente in bis zu 20-fachem, bevorzugt in etwa 10-fachem molaren Überschuss eingesetzt.

Die Reaktionskomponenten werden miteinander vermischt und bei Temperaturen oberhalb 160 °C unter ständigem Vermischen thermolytisch behandelt. Die Reaktionsdauer ist dabei erwartungsgemäss abhängig von der Reaktionstemperatur. Sie ist z.B. bei 150 °C unwirtschaftlich lang und liegt bei 160 °C etwa bei 50 h. Eine Temperaturerhöhung über 220 °C bedingt zwar eine weitere Reaktionszeitverkürzung, aber auch eine verstärkte Bildung harzartiger Nebenprodukte. Nach beendeter thermolytischer Reaktion werden das entstandene Kupferhalogenid sowie nicht umgesetztes Kupfer mittels Filtration der heissen Reaktionslösung abgetrennt. Nicht umgesetzte Naph-thoesäure wird mit Alkalibicarbonat extrahiert und Naphtholacton, Naphthylester, Naphthalin, die Halogenaromaten und die dehalogenierten Halogenaromaten werden destillativ aufgearbeitet.

Beispiele

Beispiel 1

17,2 g (0,1 Mol) Naphthoesäure-(1), 147 g (1 Mol) 1,2-Dichlorbenzol und 11,5 g (0,18 Mol) Kupfer werden 46 h bei 168°C gerührt. Nach Abtrennen von 17,8 g Kupfer(I)-chlorid durch heisse Filtration wird die Lösung mit wässriger Natriumbicarbonat-lösung extrahiert. Durch anschliessendes Ansäu-ern des wässrigen Extraktes und Filtration werden 2,58 g (15 Gew.-%) 1-Naphthoesäure zurückgewonnen.

Aus dem 1,2-Dichlorbenzolextrakt können 7,95 g 8-Hydroxi-(1)-naphthoesäure-lacton, 1,1 g Naphthalin sowie 1,73 g Naphthoe-säure-1-naphthylester gewonnen werden. Die Ausbeuten betragen demnach:

| | |
|---|---|
| 8-Hydroxy-(1)-naphthoesäure-lacton | 55% |
| Naphthalin | 10% |
| Naphthoesäure-1-naphthylester | 14% |

Beispiel 2

17,2 g (0,1 Mol) Naphthoesäure-(1), 180 g 1,2,4-Trichlorbenzol und 11,5 g (0,18 Mol) Kupfer werden 6 h bei 123°C gerührt. Das weitere Vorgehen erfolgt gemäss Beispiel 1. Bei einem Umsatz von 70% betragen die Ausbeuten:

| | |
|---|---|
| 8-Hydroxy-(1)-naphthoesäure-lacton | 47% |
| Naphthalin | 12% |
| Naphthoesäure-1-naphthylester | 10% |

Beispiel 3

17,2 g (0,1 Mol) Naphthoesäure-(1), 50 g 4-Chloro-xylol und 11,5 g (0,18 Mol) Kupfer werden 11 h unter Rühren auf 190°C erhitzt. Das weitere Vorgehen erfolgt gemäss Beispiel 1. Bei einem Umsatz von 64% betragen die Ausbeuten:

| | |
|---|---|
| 8-Hydroxy-(1)-naphthoesäure-lacton | 22% |
| Naphthalin | 6% |
| Naphthoesäure-1-naphthylester | 2% |
| Naphthoesäuremethylester | 26% |

Beispiel 4

17,2 g (0,1 Mol) Naphthoesäure-(1), und ein Gemisch aus je 50 g 1,2,3-, 1,2,4- und 1,3,5-Trichlorbenzol sowie 11,5 g (0,18 Mol) Kupfer werden 10 h bei 207°C gerührt. Das weitere Vorgehen erfolgt gemäss Beispiel 1. Bei einem Umsatz von 75% betragen die Ausbeuten:

| | |
|---|---|
| 8-Hydroxy-(1)-naphthoesäure-lacton | 49% |
| Naphthalin | 10% |
| Naphthoesäure-1-naphthylester | 5% |

Beispiel 5

20,3 g (0,05 Mol) Kupfer(II)-naphthoat-(1) werden in 80 g siedendem 1,2,4-Trichlorbenzol 4 h gerührt. Das weitere Vorgehen erfolgt gemäss Beispiel 1. Die Ausbeuten betragen bei einem Umsatz von 80%:

| | |
|---|---|
| 8-Hydroxy-(1)-naphthoesäure-lacton | 60% |
| Naphthalin | 5% |
| Naphthoesäure-1-naphthylester | 7% |

Beispiel 6

20,3 g (0,05 Mol) Kupfer(II)-naphthoat-(1) werden in 80 g siedendem 1,3,5-Trichlorbenzol 4 h gerührt. Nach Verdünnen mit 50 ml Xylol erfolgt das weitere Vorgehen gemäss Beispiel 1. Die Ausbeuten betragen bei einem Umsatz von 78%:

| | |
|---|---|
| 8-Hydroxy-(1)-naphthoesäure-lacton | 55% |
| Naphthalin | 2% |
| Naphthoesäure-1-naphthylester | 1% |

Beispiel 7

20,3 g (0,05 Mol) Kupfer(II)-naphthoat (1) werden in 50 g 1,2,4,5-Tetrachlorbenzol 2 h bei 240°C gerührt. Nach Verdünnung mit 50 ml Xylol erfolgt das weitere Vorgehen gemäss Beispiel 1. Bei einem Umsatz von 76% betragen die Ausbeuten:

| | |
|---|---|
| 8-Hydroxy-(1)-naphthoesäure-lacton | 57% |
| Naphthalin | 1% |
| Naphthoesäure-1-naphthylester | 7% |

Beispiel 8

20,3 g (0,05 Mol) Kupfer (II)-naphthoat-(1) werden in einem Gemisch aus je 30 g 1,2,3-, 1,2,4- und 1,3,5-Trichlorbenzol 8 h bei 250°C gerührt. Das weitere Vorgehen erfolgt gemäss Beispiel 1. Die Ausbeuten betragen bei einem Umsatz von 80%:

| | |
|---|---|
| 8-Hydroxy-(1)-naphthoesäure-lacton | 57% |
| Naphthalin | 3% |
| Naphthoesäure-1-naphthylester | 9% |

**Patentansprüche**

1. Verfahren zur Herstellung von 8-Hydroxi-(1)-naphthoesäurelacton aus Naphthoesäure-(1) dadurch gekennzeichnet, dass Naphthoesäure-(1) in Gegenwart der mindestens zweifachen molaren Menge an kernhalogenierten aromatischen Kohlenwasserstoffen und der zweifachen molaren Menge an Kupfer bei Temperaturen oberhalb von 160°C thermisch behandelt wird.

2. Verfahren zur Herstellung von 8-Hydroxi-(1)-naphthoesäurelacton aus Kupfer(II)-naphthoat dadurch gekennzeichnet, dass Kupfer(II)naphthoat in Gegenwart der mindestens zweifachen molaren Menge von kernhalogenierten aromatischen Kohlenwasserstoffen bei Temperaturen oberhalb von 160°C thermisch behandelt wird.

3. Verfahren nach Anspruch 1 oder 2, dadurch gekennzeichnet, dass als kernhalogenierter aromatischer Kohlenwasserstoff ein kernchloriertes Benzolderivat verwendet wird.

4. Verfahren nach Anspruch 1 oder 2, dadurch gekennzeichnet, dass die thermische Behandlung bei einer Temperatur im Bereich von 160 bis 220°C durchgeführt wird.

**Claims**

1. A process for the preparation of 8-hydroxy-1-naphthoic acid lactone from 1-naphthoic acid, characterised in that 1-naphthoic acid is heat-treated at temperatures above 160 °C in the presence of an at least double molar quantity of ring-halogenated aromatic hydrocarbons and a double molar quantity of copper.

2. A process for the preparation of 8-hydroxy-1-naphthoic acid lactone from copper(II)-naphthoate, characterised in that copper(II)-naphthoate is heat-treated at temperatures above 160 °C in the presence of an at least double molar quantity of ring-halogenated aromatic hydrocarbons.

3. A process according to Claim 1 or 2, characterised in that a ring-chlorinated benzene derivative is used as ring-halogenated aromatic hydrocarbon.

4. A process according to Claim 1 or 2, characterised in that the heat treatment is carried out at a temperature in the range of 160 to 220 °C.

**Revendications**

1. Procédé de préparation de lactone d'acide hydroxy-8 naphtoïque-1, caractérisé en ce que l'acide naphtoïque-1 est traité par voie thermique à des températures supérieures à 160 °C en présence d'une quantité au moins deux fois molaire d'hydrocarbures aromatiques halogénés dans le noyau et d'une quantité deux fois molaire de cuivre.

2. Procédé de préparation de lactone d'acide hydroxy-8 naphtoïque-1 à partir de naphtoate du cuivre(II), caractérisé en ce que du naphtoate de cuivre(II) est traité par voie thermique à des températures supérieures à 160 °C en présence d'une quantité au moins deux fois molaire d'hydrocarbures aromatiques halogénés dans le noyau.

3. Procédé selon la revendication 1 ou 2, caractérisé en ce qu'un dérivé de benzène chloré dans le noyau est utilisé comme hydrocarbure aromatique halogéné dans le noyau.

4. Procédé selon la revendication 1 ou 2, caractérisé en ce que le traitement thermique s'effectue à une température dans la plage de 160 à 220 °C.